# EUROPEAN PATENT SPECIFICATION

(11) **EP 3 970 640 B1**
(45) Date of publication and mention of the grant of the patent: **13.05.2026**
(21) Application number: 20196888.0
(22) Date of filing: 18.09.2020
(51) Int. Cl.: A61B 17/17, A61B 17/70, A61F 2/46, A61B 34/20, A61B 90/00

(54) **SURGICAL DEVICE WITH A MOVABLE TRACKER**
CHIRURGISCHE VORRICHTUNG MIT BEWEGLICHEM NACHFÜHRER
DISPOSITIF CHIRURGICAL AYANT UN SUIVEUR MOBILE

(43) Date of publication of application: 23.03.2022
(73) Proprietor: Stryker European Operations Limited, Carrigtwohill, Co. Cork T45 HX08 (IE)
(72) Inventor: HERRMANN, Florian, 77963 Schanau (DE); SCHÖPP, Hans, 79110 Freiburg (DE); PULEVA, Nikolina, 79106 Freiburg (DE)
(74) Representative: Röthinger, Rainer

(56) References cited:
- EP-A1- 1 415 609
- US-A1- 2010 039 506
- US-A1- 2012 059 472
- US-A1- 2012 143 050
- US-A1- 2013 023 994
- US-A1- 2016 175 064
- US-A1- 2018 085 233
- US-A1- 2019 038 366

## Description

### Technical Field

The present disclosure generally relates to the field of computer-assisted surgical navigation using optical or non-optical tracking technologies. In particular, a surgical device with a movable tracker, a system comprising the surgical device, a computer-implemented method involving the surgical device, and a computer program product are presented.

### Background

Many surgical procedures benefit from determination of a state, a position, or an orientation of a surgical device in an operating room. To this end, surgical devices are equipped with trackers trackable by a surgical navigation system with tracking capabilities (e.g., a navigation camera). A result of the tracking may then be visualized by the surgical navigation system, for example relative to image data of a patient treated by the surgical device.

In a typical application of a surgical navigation system, the surgical device and the patient are each associated with a tracker, wherein image data previously obtained by, for example, a computer tomography (CT) scan are initially registered with a pose of a patient tracker. By then continuously tracking the patient and the surgical device, the surgical navigation system can determine a spatial relationship between the surgical device and the three dimensional image data. The determined spatial relationship can, for example, be displayed on a screen, helping the surgeon operate the surgical device relative to the patient.

Typical surgical devices that rely on optical trackers for navigation comprise a distal end configured to enter the body of a patient and a proximally arranged optical tracker configured for line of sight tracking by the navigation camera. Some of those surgical devices further comprise an operation member that is operable by a surgeon to actuate a mechanism provided in a region of the distal end. Evidently, actuation of this mechanism is difficult to track because line of sight tracking cannot be employed during a surgical intervention when the distal end has entered the patient body.

Document US 2019/038366 A1 discloses techniques for determining the shape of a surgical implant and navigating the surgical implant during surgery. The shape of the surgical implant is determined during surgery and a graphical representation of the surgical implant accurately depicting its shape are displayed and overlaid on graphical depiction of a target anatomy of a patient.

Document US 2013/023994 A1 discloses an expandable fusion device capable of being installed inside an intervertebral disc space to maintain normal disc spacing and restore spinal stability, thereby facilitating an intervertebral fusion. In one embodiment, the fusion device includes a body portion, a first endplate, and a second endplate, the first and second endplates capable of being moved in a direction away from the body portion into an expanded configuration or capable of being moved towards the body portion into an unexpanded configuration. The fusion device is capable of being deployed and installed in both configurations.

Document US 2012/059472 A1 discloses an expandable fusion device capable of being installed inside an intervertebral disc space to maintain normal disc spacing and restore spinal stability, thereby facilitating an intervertebral fusion. In one embodiment, the fusion device includes a central ramp, a first endplate, and a second endplate, the central ramp capable of being moved in a first direction to move the first and second endplates outwardly and into an expanded configuration.

Document US 2018/085233 A1 discloses a spinal implant defining an axis. A first image guide is connected with a member and oriented relative to a sensor to communicate a signal representative of a position of the member. A second image guide is connected with the member and oriented to represent an angle measuring a second orientation of the axis relative to a first orientation.

Document EP 1 415 609 A1 disclose an optical tracking system. The tracking system comprises a camera system including at least two spatially separated cameras capable of viewing the clinical field of view to provide camera data in a first coordinate system defined by the camera system; an instrument comprising an optically detectable object that is detectable by the camera system to provide instrument data representative of the position of the instrument in the first coordinate system; a headband positionable on the head of a surgeon, the headband including a display viewable by the surgeon and an optically detectable array and that is detectable by the camera system to provide headband data representative of the position of the headband in the first coordinate system; data storage comprising one of CT and MRI image data representative of the anatomy of the patient received from one of a CT and MRI imaging machine; and a computer to accept the camera data, the instrument data, the headband data, and the image data, and being programmed to transform the image data, the camera data, the headband data, and the instrument data into a second coordinate system, thereby generating tracking data representative of the position of the instrument and the headband in relation to the anatomy of the patient.

Document US 2012/0143050 A1 discloses a reference foil. The reference foil comprises an unsymmetric marker foil device which advantageously includes spatially separated pieces of a marker material foil in unsymmetric arrangement and/or at least one unsymmetric integral piece of the marker material foil.

Document US 2010/039506 A1 discloses a navigation system. The navigation system includes a display monitor, a CPU, and a camera, wherein the camera is mounted to a back side of the display monitor to form a monitor unit. One or more reference units are placed on a body while acquiring an image data set, and are tracked during a surgical operation by the monitor unit to register and correlate a position of a visual image of an exterior surface of the body with the image data set including information concerning internal structures of the body. The image data set is displayed on the display monitor superimposed in registration over the visible image of the exterior of the body with an angle of view and aperture in accordance with the actual position of the camera, whereby the display monitor displays the internal structures corresponding to the line-of-sight of the camera and the observer.

Document US 2016/175064 A1 discloses a medical navigation marker device. The medical navigation marker device comprises a light reflector, characterised in that the reflector features a marker pattern having points in more than one spatial direction. The marker pattern comprises at least one moire pattern which points in more than one spatial direction and the marker pattern comprises a face identification pattern which identifies the face of the marker being viewed from a particular spatial direction. The invention also relates to a combination of a medical marker device and a medical navigation system which uses the marker device as a spatial position and/or orientation marker, and to the use of a medical marker device for providing guidance to a user of a medical navigation system by evaluating the spatial position and/or orientation information provided by the marker device.

### Summary

There is a need for a trackable surgical device that extends the tracking capabilities of hitherto known surgical navigation systems.

The invention is defined in the appended independent claims. Preferred embodiments are recited in the dependent claims.

According to a first aspect, a surgical device is presented that comprises a shaft defining a longitudinal axis, wherein the shaft has a first shaft portion configured to be inserted into a patient and a second shaft portion configured to be located outside the patient when the first shaft portion is inserted in the patient. The surgical device further comprises an actuation member actuatable relative to the first shaft portion and an operation member operable relative to the second shaft portion, the operation member being configured to actuate the actuation member when being operated. Further, the surgical device comprises a first tracker coupled with the operation member so that operation of the operation member causes the first tracker to rotate relative to the longitudinal axis.

The operation of the operation member causes the first tracker to rotate relative to the longitudinal axis. In another variant, an operation of the operation member may cause a movement of the first tracker that combines a rotation with a translation (e.g., along the longitudinal axis).

According to one variant, there may exist a predefined relationship between a movement of the first tracker on the one hand and an actuation movement of one of the actuation member and an actuatable implant coupled to the actuation member on the other hand. The movement of the first tracker is a rotational movement. The actuation movement of one of the actuation member and the actuatable implant detachably couplable to the actuation may be a movement relative to the first shaft portion. For example, the actuation movement of the actuation member may be a rotational movement around the longitudinal axis. The rotational movement may occur clockwise or counterclockwise.

The predefined relationship may associate a change of a rotational state of the first tracker resulting from its rotation with a change of a dimension or state resulting from the actuation movement. The associated change of rotational state of the first tracker is a change of an angular position of the first tracker around the longitudinal axis resulting from a rotational movement. The change of dimension or state may relate to any one of the actuation member and the actuatable implant detachably couplable to the actuation member. The change of dimension may comprise a change in height, length or width of at least a part of the actuation member and/or the actuatable implant coupled to the actuation member, or any combination of these changes. The change of state may be of a discrete nature (e.g., extended/retracted or on/off).

The predefined relationship may be a functional relationship. Using this functional relationship, the surgical navigation system may computationally translate a detected rotational movement (e.g., an angle of rotation resulting from the change of the angular position) into an associated change of dimension or state. The change of dimension or state of the actuation member or the actuatable implant may visually be presented to the surgeon, for example relative to image data of a patient.

According to one variant, the operation member is rotatable around the longitudinal axis. In other variants, the operation member is movable in a translatory manner or rotatable around an axis different from the longitudinal axis.

The operation member may be a handle (e.g., realized as a rotatable knob or lever). The operation member may be operated manually or automatically. The operation member may be configured to interact with a gear mechanism configured to translate the operation movement applied to the operation member into the actuation movement of the actuation member or the actuatable implant coupled to the actuation member. This translation may take place between a rotatory operation movement and a translatory actuation movement, or vice versa, or between different rotatory or translatory movements.

The first tracker may be rigidly coupled or configured to be rigidly coupled to the operation member. In this variant, a rotational movement of the operation member around the longitudinal axis results in a corresponding rotational movement of the first tracker. The rigid coupling between the first tracker and the operation member may be such that the first tracker can be detached from the surgical device.

Further, the first tracker is an optical tracker (e.g., for being tracked in the infrared and/or the visible spectrum). The first tracker comprises a plurality of markers (e.g., at least 3, 4, 5 or 6 markers and up to 10 or 14 or more markers). These markers are optically-detectable markers, such as optically-passive markers (e.g., reflectors) or optically-active markers (e.g., Light Emitting Diodes, LEDs). The optically-passive markers may have a generally flat shape that may optionally be curved around the longitudinal axis. In other variants, the optically-passive markers may have a spherical shape.

The markers of the first tracker are arranged in a pattern that defines, from a given viewing direction, distinctive arrangements of the markers in different rotational states of the first tracker relative to the longitudinal axis. As such, at least some of the markers are spaced apart from each other in a circumferential direction around the longitudinal axis. The markers may be trackable by a sensor of a surgical navigation system. The sensor may be an optical sensor. The optical sensor may be a mono camera or stereo camera.

The surgical device may comprise a second tracker rigidly coupled or configured to be rigidly coupled to the shaft. The second tracker may be an optical tracker (e.g., for being tracked in the infrared and/or the visible spectrum). The second tracker may comprise a plurality of optical tracking elements such as reflective spheres or active LEDs. The second tracker may comprise 3, 4 or 5 optical tracking elements (that, optionally, need all to be tracked simultaneously for proper navigation). The optical tracking elements may be trackable by an optical sensor of a surgical navigation system. This optical sensor may further be configured to track the markers of the first tracker when same is configured as an optical tracker with optically-detectable markers.

According to one variant, the operation member may be movable relative to the shaft in an axial direction relative to the longitudinal axis from a predefined first axial position to a predefined second axial position. In one example, the movement of the operation member between the predefined axial positions may be translated into a rotational movement of the actuation member. The predefined axial positions may relate to predefined distinct actuation states of the actuatable implant (e.g., extracted or retracted).

The surgical device may be configured such that an axial movement of the operation member relative to the shaft leads to an axial movement of the first tracker relative to the second tracker. In one example, this axial movement may be translated into a rotational movement of the actuation member. The rotational movement of the actuation member may be derived based on tracking a change of the distance between the first tracker and the second tracker.

Operation of the operation member may cause a rotation of the actuation member relative to the first shaft portion. In case the first shaft portion is rigidly coupled to the second shaft portion, the actuation member will also rotate relative to the second shaft portion. The rotation of the actuation member may correspond to a rotation of the operation member around the longitudinal axis (e.g., in terms of an angular change), especially when the operation member is rigidly coupled with the actuation member.

According to one variant, the shaft defines a passage and the operation member is coupled to the actuation member via a coupling section extending into the passage defined by the shaft. The passage may be defined by a cylindrical cavity within the shaft configured to removably receive the coupling section and, optionally, the actuation member. The passage may extend coaxially to the longitudinal axis of the shaft. In some variants, the shaft takes the form of a tube, wherein a distal section of the tube forms the first shaft portion and a proximal section of the tube forms the second shaft portion.

The coupling section may comprise an inner and an outer driver configured to be movable relative to each other in an axial direction relative to the longitudinal axis from a predefined first axial position to at least a predefined second axial position. The mutual relationship of the inner and outer driver may be defined in a radial direction relative to the longitudinal axis as a radially inner and a radially outer position.

At least one of the inner driver and the outer driver may be configured to include or cooperate with the actuation member. For example, the actuation member may comprise an inner part and an outer part, which are (e.g., integrally) coupled to and move correspondingly to the inner and outer driver, respectively. This implementation may enable different actuation modes based on the positions of the inner and the outer driver of the coupling section relative to each other (e.g., only the inner part acts as actuator, only the outer part acts as actuator, or both parts act as actuators).

The surgical device may further comprise a drive member coupled to at least one of the inner driver and the outer driver. The drive member may be configured to be movable relative to the shaft in an axial direction relative to the longitudinal axis from a predefined first axial position to at least a predefined second axial position, so that a movement of the drive member from the predefined first axial position to the at least predefined second axial position results in a change of position of the inner driver relative to the position of the outer driver (e.g., a movement of the drive member from a distal to a more lateral position results in a movement of the inner driver to a more lateral position relative to the outer driver and vice versa). In particular, the drive member may have three predefined positions along the longitudinal axis (e.g., so as to realize the three actuation modes mentioned above).

In some implementations, the drive member may be rigidly coupled with the first tracker. In other implementations, the first tracker may constitute the drive member or a part thereof. In still further implementations, a dedicated third tracker may be provided for the drive member (e.g., the third tracker may be rigidly coupled to the drive member). The third tracker may be an optical tracker (e.g., for being tracked in the infrared and/or the visible spectrum). In these cases, operation of the drive member may be tracked also (e.g., relative to the second tracker).

The first shaft portion may be configured to cooperate with a first portion of an implant. The actuation member may be configured to cooperate with a second portion of the implant, wherein the second portion is movable relative to the first portion for a change of an actuation state of the implant. The shaft may be configured to detachably receive the implant for placement of the implant in the body of the patient. The implant may be detached inside the body of the patient, in particular following the actuation movement.

The first tracker is an optical tracker and comprise a body. The first tracker may comprise a cover layer for the body and one or more optically detectable markers arranged between the body and the cover layer. The cover layer may be optically transparent (e.g., in the infrared and/or visual spectrum) at least in one or more regions where the one or more markers are located. One or more optically detectable markers are located on or within the body. The cover layer may be configured for a transmission of light of a specific wavelength, which can be reflected or emitted by the markers and tracked by an optical sensor. The cover layer may be attached to the body detachably (e.g., for cleaning, sterilization or maintenance purposes) or non-detachably.

The body of the first tracker is arranged or configured to be arranged co-axially relative to the longitudinal axis of the shaft. The body has a substantially cylindrical shape with a cylinder axis. The body may in particular have a drum shape with a central opening. In other variants, the body may have a certain number of distinct sides, such as four in the case of a cube. The first tracker may be detachable from the surgical device (e.g., for cleaning or sterilization purposes).

According to a second aspect, a system comprising a surgical device according to the first aspect and an implant configured to be actuated by the actuation member from a first actuation state to a second actuation state is provided. The actuation may be a discrete or a continuous actuation.

According to one variant, the implant is configured to change at least one dimension when being actuated from the first actuation state to the second actuation state. The change of the at least one dimension may be an increase or a decrease of the at least one dimension (e.g., height, width or length).

Further, at least a part of the implant may be configured to extend relative to the first shaft portion when the implant is actuated from the first actuation state to the second actuation state. Alternatively, or in addition, at least a part of the implant may be configured to retract when the implant is actuated from the second actuation state to the first actuation state.

According to an example useful for understanding the invention, a computer-implemented method for determining an actuation performed with a surgical device is provided. The surgical device comprises a shaft defining a longitudinal axis, the shaft having a first shaft portion configured to be inserted into a patient and a second shaft portion configured to be located outside the patient when the first shaft portion is inserted in the patient, an actuation member actuatable relative to the first shaft portion, an operation member operable relative to the second shaft portion, the operation member being configured to actuate the actuation member when being operated and a first optical tracker coupled with the operation member so that operation of the operation
member causes the first tracker to rotate relative to the longitudinal axis. The first optical tracker comprises a plurality of markers arranged in a pattern that defines, from a given viewing direction, distinctive arrangements of the markers in different rotational states of the first optical tracker relative to the longitudinal axis. The method comprises determining a rotational state of the first optical tracker and determining, from at least the rotational state of the first optical tracker, an actuation of at least one of the actuation member and an implant detachably couplable to the actuation member.

The rotational state of the first tracker that is determined according to the third aspect is a rotational state of the first tracker around the longitudinal axis defined by the shaft of the surgical device.

Determining the actuation of the actuation member may comprise determining an actuation movement or an actuation state thereof. The actuation state may be determined out of a predefined set of two or more actuation states (e.g., on/off or extended/retracted). The actuation movement may be determined as an angular or translatory distance (e.g., in the form of a continuous parameter).

The position of the first tracker is determined by tracking a distinctive arrangement of markers of the first tracker from a given viewing angle, the distinctive arrangement representing a rotational state relative to the longitudinal axis. The determined actuation of the actuation member may be further used to determine a change of a dimension of one of the actuation member and an actuatable implant connected to the actuation member.

According to one variant, the method may comprise determining an actuation state of the actuation member resulting from the actuation relative to a known initial state prior to the actuation. The initial state may be known from a manual or automatic determination prior to the insertion of the implant into the body of a patient, or otherwise. This analogously applies to an actuation state of an actuatable implant.

The position of the first tracker may be determined relative to a second tracker having a fixed relationship relative to the shaft by tracking the first and second trackers (e.g., with an optical sensor in the case the trackers are optical trackers). The second tracker may have an angularly fixed relationship relative to the longitudinal axis. Therefore, both trackers may be tracked simultaneously.

Further, the method may comprise visualizing the actuation of the actuation member on a display. Visualizing the actuation of the actuation member may be performed in real-time relative to an image of the patient. The image of the patient may be generated based on computer tomographic (CT) image data.

According to a third aspect, a computer program product with program code for carrying out the method according to the example useful for understanding the invention when executed by a processor is provided. The processor may be a part of a computing system commonly used for computer assisted surgery.

According to another example useful for understanding the invention, a tracker, in particular for use with the surgical device described herein, is provided. The tracker is an optical tracker and comprises a body on which or within which one or more optically-detectable markers are arranged. The body and markers are covered by a cover layer that is optically transparent at least in a region of the one or more markers. The cover layer may be configured for a transmission of light of a specific wavelength (e.g., infrared and/or visible light), which can be reflected by the optical elements and tracked by an optical sensor.

The cover layer may comprise an elastic material so it can easily be positioned around the body or removed from the body. The cover layer may comprise at least two different material layers, wherein an inner layer is relatively softer and an outer layer is relatively harder. In this example, the cover layer does not damage the optically-detectable markers that are located on or within the tracker body and, at the same time, is robust against damages, e. g., mechanical damages like scratches, to its outer surface. The cover layer may be smooth on its outside so that it can easily be cleaned and sterilized.

### Brief Description of the Drawings

Further details, advantages and aspects of the present disclosure will become apparent from the following embodiments taken in conjunction with the drawings, wherein:
- Fig. 1A: shows a perspective view of an embodiment of a surgical device according to the present disclosure, comprising a first and a second part of the device in a decoupled state;
- Fig. 1B: shows a perspective view of the embodiment of Fig. 1A, wherein the two parts are in a coupled state;
- Fig. 2A: shows the first device part of the embodiment of Figs. 1A and 1B;
- Fig. 2B: shows an embodiment of an optical tracker comprising a body and a cover layer in an exploded view;
- Fig. 2C: shows the optical tracker of Fig. 2B in an assembled state;
- Fig. 2D: shows an embodiment of the first device part with an alternative arrangement of the operation member and the optical tracker;
- Fig. 2E: shows an embodiment of the first device part with a drive member and a coupling section comprising an inner and outer driver;
- Figs. 3A: shows a front view of an outer driver of a coupling section of the first part of the surgical device presented herein;
- Fig. 3B: shows a front view of the outer driver and an inner driver of the coupling section of the first part of the surgical device;
- Fig. 4: shows a perspective view of the second device part of the embodiment of Figs. 1A and 1B;
- Figs. 5A, B: show perspective and side views of a system embodiment comprising the surgical device in its coupled state as shown in Fig. 1B and an actuatable implant;
- Figs. 6A - C: show different views of an actuatable implant;
- Fig. 6D: shows a schematic representation of a drive member in a first position and an implant actuated for posterior expansion or retraction;
- Fig. 6E: shows a schematic representation of a drive member in a second position and an implant actuated for lordotical expansion or retraction;
- Fig. 6F: shows a schematic representation of a drive member in a third position and an implant actuated for a symmetric height expansion or retraction;
- Fig. 7: shows a flow diagram of a method embodiment for determining an actuation of an actuation member of a surgical device;
- Fig. 8: shows a schematic representation of a computer program product with code executable by a processor;
- Fig. 9: shows a schematic representation of a surgical navigation system embodiment with an optical sensor used for tracking actuation of the actuation member and implant;
- Fig. 10: shows an example visualization of a part of the surgical device and of the implant during a spinal treatment surgery; and
- Figs. 11A, B: show schematic representations of a visualization of a part of the surgical device and the actuatable implant during a spinal surgery.

### Detailed Description

In the following description, exemplary embodiments will be explained with reference to the drawings. The same reference numerals will be used to denote the same or similar structural features.

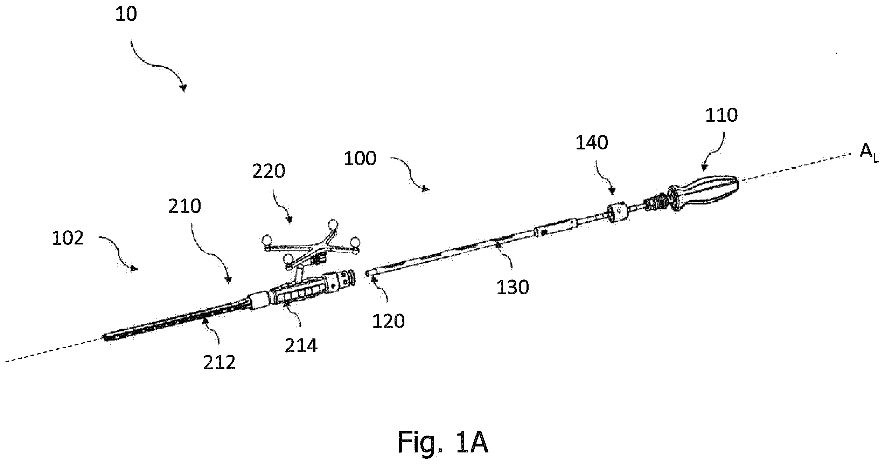

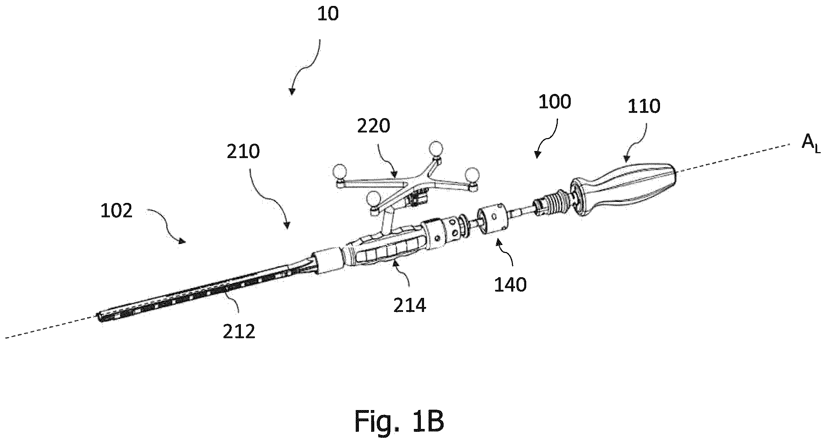

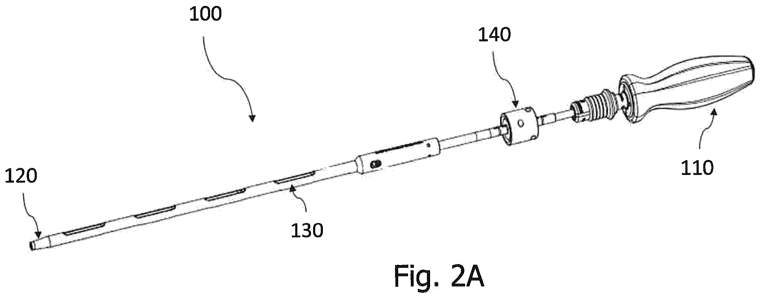

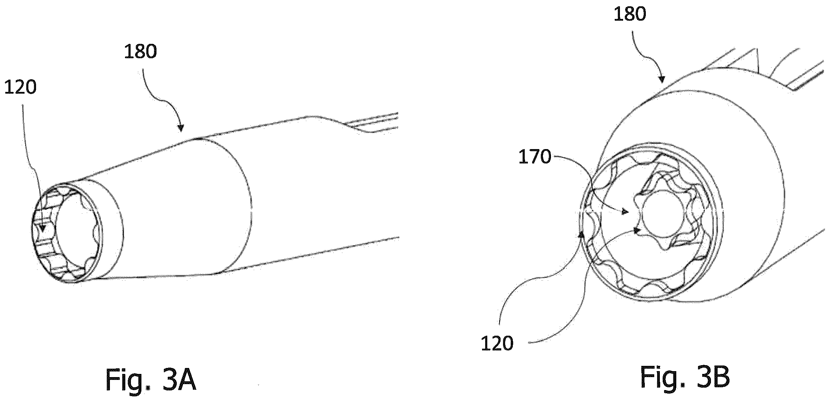

Figs. 1A and 1B show an embodiment of a surgical device 10 according to the present disclosure. The surgical device 10 comprises two device parts 100, 102 that can be coupled with each other. Fig. 1A, shows the two device parts 100, 102 in a decoupled state, and Fig. 1B shows the two device parts 100, 102 in a coupled state. The two device parts 100, 102 each have a longitudinal extension along a longitudinal axis A_{L}. The second device part 102 has a generally tubular configuration and is configured to coaxially receive the first device part 100. The individual components of the surgical device 10 and their functions will be described in greater detail below with reference to Figs. 2A to 2E and 3.

Fig. 2A illustrates the first device part 100 of the embodiment shown in Figs. 1A and 1B. The first device part 100 has a rod-like shape and comprises a operation member 110 at its proximal end. In the present embodiment, the operation member 110 is a handle exemplarily having a knob-like configuration.

The operation member 110 is rigidly coupled to an actuation member 120 provided at a distal end of the first device part 100. In more detail, a rigid coupling section 130 extends between and interconnects the operation member 110 and the actuation member 120. The operation member 110 is operable to actuate the actuation member 120. Rotation of the operation member 110 around the longitudinal axis A_{L} leads to a corresponding rotation of the actuation member 120. The rotation around the longitudinal axis A_{L} can be clockwise or counter-clockwise.

In other examples a gear mechanism may functionally be provided between the operation member 110 and the actuation member 120. The gear mechanism may enable that a given rotational speed of the operation member 110 results in a different rotational speed of the actuation member 120. In one example, the gear mechanism may function as a reduction gear for fine adjustments. For example, the rotational speeds of the operation member 110 and the actuation member 120 have a ratio of 2:1 (e.g., a rotation of the operation member 110 of 2° results in an rotation of the actuation member of 1°).

The first device part 100 of the surgical device 10 further comprises a first tracker 140. The first tracker is an optical tracker 140 that can be tracked in the infrared or visible spectrum by a suitable optical tracking system. Also the further trackers described herein are configured as such optical trackers. It will be appreciated that the present disclosure is not limited to optical trackers and an optical tracking system. Rather, one or more of the trackers described herein could also be configured as electromagnetic trackers (each including, e.g., one or more tracking coils) or as ultrasound trackers to be tracked by a suitable electromagnetic or ultrasound tracking system.

With continued reference to Fig. 2A, optical tracker 140 is detachably coupled to the first device part 100 in a rotationally fixed manner, for example via a plug-in connection. In other examples, the optical tracker 140 may be clamped to the first device part 100. The optical tracker 140 is rigidly (but detachably) coupled to the operation member 110 so that a rotation of the operation member 110 around the longitudinal axis A_{L} results in a corresponding rotation of the optical tracker 140. Referring to the example wherein the coupling section 130 comprises a gear, the optical tracker 140 may be located on either functional side of the gear.

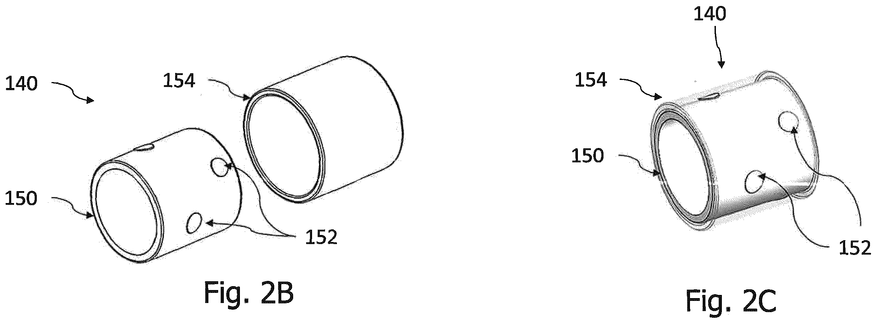

The optical tracker 140 shown in Fig. 2A is also shown in Figs 2B and 2C in greater detail. As illustrated therein, the optical tracker 140 comprises a body 150, which has a substantially hollow-cylindrical shape with a cylinder axis that is arranged co-axial to the longitudinal axis A_{L} when attached to the coupling section 130. The body 150 comprises a plurality of optically-detectable markers 152 at its outer surface. The optically-detectable markers 152 are located on the body 150 and circumferentially arranged in a pattern that defines, from a given viewing direction, distinctive arrangements of the optically-detectable markers 152 in different rotational states relative to the longitudinal axis A_{L}. The optically-detectable markers 152 are flat passive markers and comprise a reflective coating configured to reflect a specific wavelength, e.g., infrared-(IR-) light.

The optical tracker 140 further comprises a cover layer 154. The cover layer 154 is transparent (e.g., in the infrared spectrum and/or in the visible spectrum), so that the optically-detectable markers 152 are protected against damages while still being optically-detectable when the tracker body 150 is covered by the cover layer 154, as shown in Fig. 2C. In other examples, the cover layer 152 is transparent only in regions where the optically-detectable markers are located. The cover layer 154 shown in Fig. 2B is configured to be detachably attached to the tracker body 150. A detachable cover layer 154 enables, for example, cleaning, sterilization and maintenance of the body and the optically-detectable markers 152 located on the body 150. In other examples, the cover layer 154 is fixedly attached to the body 150.

In another variant not illustrated in the drawings, the optically-detectable markers 152 may be located on an inner surface of the cover layer 152. For example, the cover layer 154 may have recesses on its inner surface in which the optically-detectable markers 152 are located.

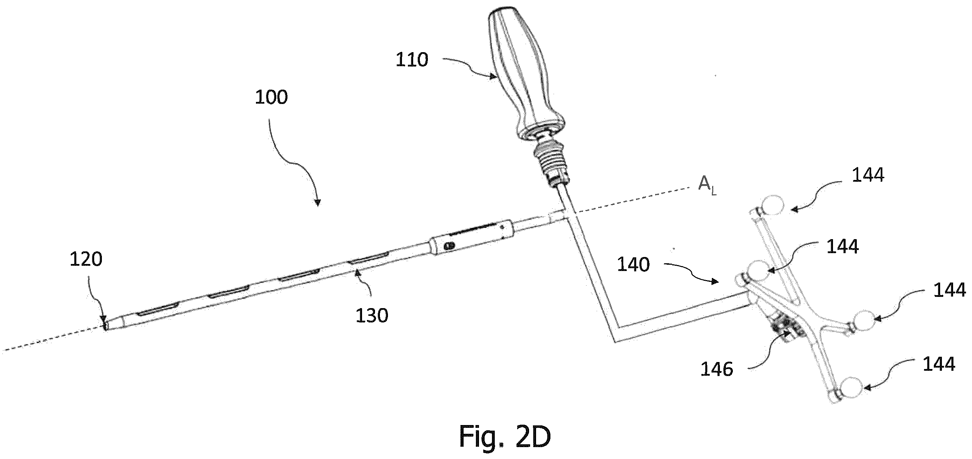

Fig. 2D shows an embodiment of the first device part 100 with an alternative arrangement of the operation member 110 and the first optical tracker 140. In this embodiment, the operation member 110 extends perpendicularly to the longitudinal axis A_{L} and the first optical tracker 140 is rigidly attached to the first device part 100 and located opposite to the operation member with regard to the longitudinal axis A_{L}. The first optical tracker 140 comprises four optical tracking elements 144 arranged on a cross-shaped mount 146 at the end of each arm. The optical tracking elements 144 are realized as passive tracking elements in the form of spheres having a reflective coating. An optical sensor (e.g., a mono camera or stereo camera, not shown in Fig. 2D) may track the movement of the optical tracking elements 144, and a corresponding rotation of the surgical device 10 can be derived computationally based on the tracked movement.

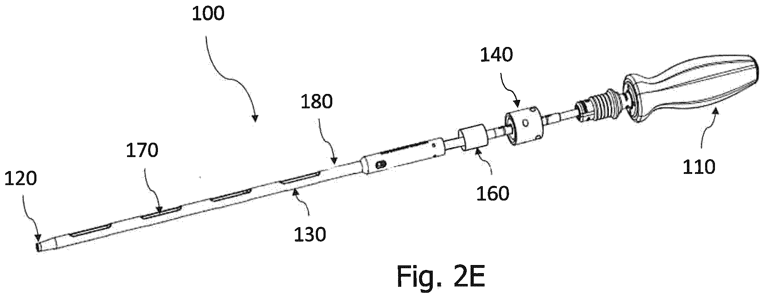

Fig 2E shows a variant of an embodiment of the first device part 100 with a drive member 160 and the coupling section 130. The coupling section 130 comprises a radially inner and a radially outer driver 170, 180 (see also Fig. 3B). The radial direction is defined relative to the longitudinal axis A_{L}. The drive member 160 is coupled to the radially inner driver 170 and movable relative to the radially outer driver 180 such that a movement of the drive member 160 results in a corresponding movement of the inner driver 170 relative to the outer driver 180. The operation member 110 is coupled to the inner driver 170 and the outer driver 180 in a rotationally fixed manner. It should be noted that in some embodiments, the axial positions of the drive member 160 and the optical tracker 140 may be switched such that the drive member 160 is closer to the operation member 110 than the optical tracker 140.

The drive member 160 has at least two, in particular three predefined positions along the longitudinal axis A_{L}. Consequently, also the inner driver 170 has at least two, in particular three predefined positions relative to the outer driver 180. In this embodiment, the actuation member 120 is provided by a combination of an end region of the inner driver 170 and an end region of the outer driver 180.

An axial movement of the inner driver 170 selectively controls an engagement of an implant part by the actuation member 120 as schematically shown in Figs. 3A and 3B. For example, in a first, or retracted, axial position (as shown in Fig. 3A) the inner driver 170 may be disengaged from the implant part. In this first position, an operation of the operation member 110 may cause an implant comprising the implant part to expand posteriorly. In a second, or extended, axial position (not shown) the outer driver 180 may be disengaged from the implant part. In this second position, an operation of the operation member 110 may cause the implant comprising the implant part to expand lordotically. In the retracted axial position, the drive member 160 may be closer to the operation member 110 than in the extended axial position. In a third predefined position (as shown in Fig. 3B), both the inner driver 170 and the outer driver 170 may be in engagement with the implant part. In this third position, an operation of the operation member 110 may cause the implant comprising the implant part to expand its height symmetrically.

In one embodiment, the functions of the drive member 160 and the first optical tracker 140 may be combined in one component (not shown) so as to reduce the number of components of the surgical device 100 while maintaining the functionality as described herein. For example, the drive member 160 may be configured to additionally function as the first optical tracker 140. In this embodiment, the drive member 160 may comprise a body, a cover layer for the body and one or more optically-detectable markers arranged between the body and the cover layer. Further, the drive member 160 may be coupled to the operation member 110 in such a way that operation of the operation member 110 may cause the drive member 160 to rotate relative to the shaft around the longitudinal axis A_{L}.

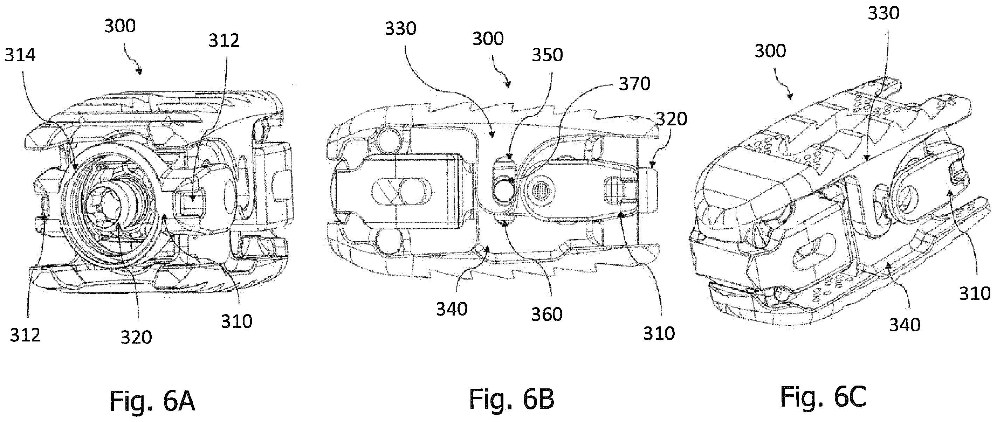

Fig. 3A shows a front view of the outer driver 180. The tip of the outer driver 180 is configured as an outer part of the actuation member 120 to cooperate with an implant that is to be actuated by the surgical device 10. The implant will be described in greater detail with reference to Figs. 6A to C below.

The tip of the outer driver 180 shown in Fig. 3A has a substantially cylindrical shape, wherein the inner surface of the tip has an actuation profile configured to cooperate in a torque proof manner with a first portion of the implant (i.e., such that the first implant portion cannot rotate relative to the outer driver 180). In more detail, the inner tip surface has an undulating profile in a circumferential direction, but other profiles such as an inner square would serve the same purpose.

Fig. 3B shows a front view of the outer driver 180 and the inner driver 170. As becomes apparent from Fig. 3B, a tip of the inner driver 170 is configured as an inner part of the actuation member 120 and comprises an actuation profile configured to cooperate in a torque proof manner with a second portion of the implant (i.e., such that the second implant portion can rotate relative to the outer driver 180 when the actuation member 120 is rotated). In more detail, the tip of the inner driver 170 has an outer surface with an undulating profile in a circumferential direction, but other profiles such as an outer square would serve the same purpose.

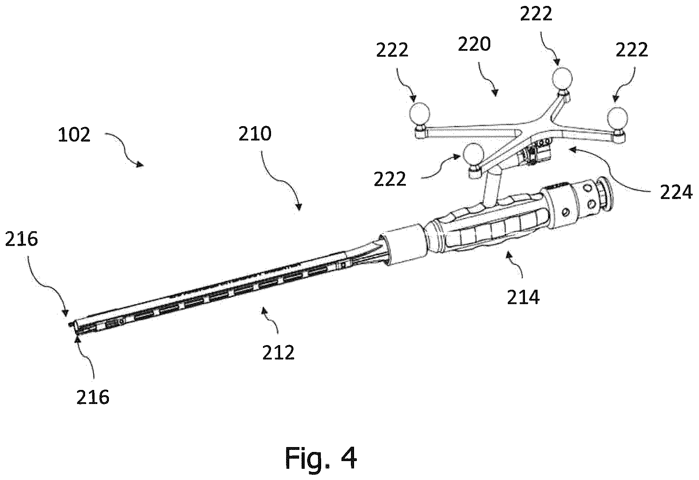

Fig. 4 shows the second device part 102 of the surgical device 10 of Figs. 1A and 1B. The second device part 102 comprises a shaft 210 having a distal first shaft portion 212 configured to be inserted into a patient and a proximal second shaft portion 214 configured to be located outside the patient when the first shaft portion 212 is inserted in the patient. The shaft 210 has a generally tubular configuration such that it defines a passage through the first shaft portion 212 and the second shaft portion 214 to coaxially receive the first device part 100 (see Fig. 1B).

The first shaft portion 212 comprises two actuatable claws 216 located on opposite sides at its distal tip and configured to detachably grip an implant (not shown in Fig. 4, see Figs. 6A to 6C below) for temporarily coupling the implant to the first shaft portion 212. The claws 216 are configured to be movable substantially perpendicularly relative to the longitudinal axis A_{L} between a closed position in which the implant is gripped and an opened position in which the implant is not gripped. The claws 216 may be coupled to an operating mechanism. A movement of the operating mechanism in a direction towards the implant opens the claws 216 and the gripped implant is released. A movement in the opposite direction closes the claws 216 and the implant is gripped by the claws 216.

The second device part 102 further comprises a handle portion at its proximal end. A second optical tracker 220 is rigidly but detachably coupled to the second shaft portion 214 in the region of this handle portion. Due to the rigid coupling of the second optical tracker 220 to the second shaft portion 214 of the second device 123 102 and the rigid coupling of the first optical tracker 140 to the first device part 100, both trackers 140, 220 move relative to each other when the two device parts 100, 102 move to each other. As such, the two trackers 140, 220 permit a determination of a relative movement between the two device parts 100, 102 by a surgical navigation system with optical tracking capabilities.

In the example of Fig. 4, the optical tracker 220 comprises four optical tracking elements 222 arranged on a cross-shaped mount 224 at the end of each arm. The optical tracking elements 222 are realized as passive tracking elements in the form of spheres having a reflective coating.

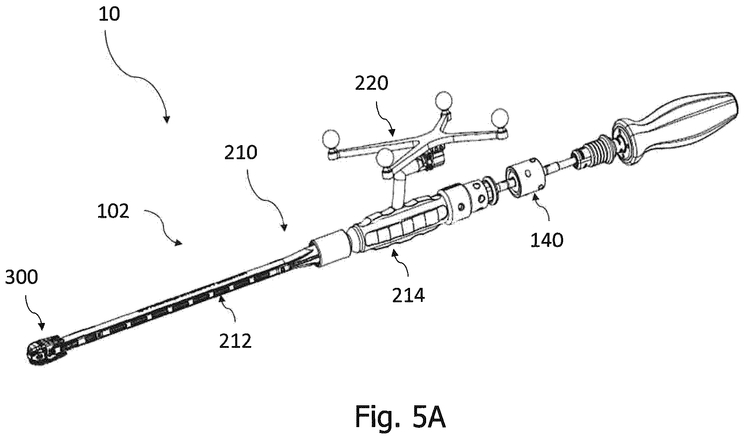

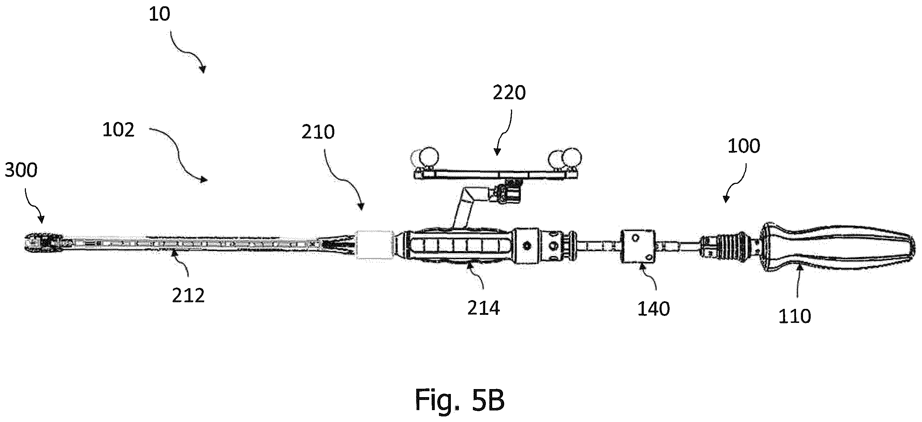

Figs. 5A and 5B show two different views of a system comprising the surgical device 10 in the coupled state as shown in Fig. 1B and an actuatable implant 300 detachably coupled thereto. In more detail, the implant 300 is held by the claws 216 to the distal end of the second shaft portion 212. The implant 300 is configured to be actuated by the actuation member 120 (as will be described in greater detail below) when the operation member 110 is operated by one hand while the shaft 210 is held stationary by the other hand.

As will be appreciated, a rotation of the operation member 110 around the longitudinal axis A_{L} results in a corresponding rotation of the actuation member 120 and the first optical tracker 140. Since the shaft 210 with the second optical tracker 220 is kept stationary, the first optical tracker 140 rotates relative to the second optical tracker 220, so that this relative rotation is detectable by an optical sensor of a surgical navigation system. Since the rotation of the actuation member 120 around the longitudinal axis A_{L} actuates the actuatable implant 300 coupled to the actuation member 120, and since the rotation of the actuation member 120 corresponds to the rotation of the first optical tracker 140, the rotational movement of the first optical tracker 140 has a predefined relationship with an actuation movement of the actuatable implant 300. In one example, this predefined relationship is given by a change of a dimension of the actuatable implant 300 in relation to an rotation angle covered by the rotational movement of the first optical tracker 140 (e.g., mm/°). The relationship could also be given in relation to a change of a discrete (e.g., binary) actuation state of the implant 300 (e.g., retracted/extended) or any combination of a dimensional change and a state change of the implant 300 in relation to the rotation angle covered by the rotational movement of the operation member 110 and, thus, first optical tracker 140.

In one example, if the rotation angle covered is greater than 359°, the number of full turns can be counted automatically (e.g., by a computing system connected to the optical sensor of the surgical navigation system). In another example, the first optical tracker 140 is configured to change its position along the longitudinal axis A_{L} every full turn, so that the number of full turns can be measured (e.g., derived by the distance between the first optical tracker 140 and the second optical tracker 220).

Figs. 6A to 6C show detailed views of the actuatable implant 300. In Fig. 6A, the actuatable implant 300 is shown in a rear view with a focus on first and second portions 310, 320 of the implant 300 configured to cooperate with the first shaft portion 212 and the actuation member 120, respectively.

The first portion 310 of the implant 300 comprises two notches 312 on opposite sides of the first portion 310. The notches 312 are configured to cooperate with corresponding claws 216 of the surgical device 10 (not shown in Figs 6A to 6C; see for example Fig. 4) to rigidly and releasably couple the first portion 310 of the implant 300 to the first shaft portion 212.

The center of the first portion 310 of the implant 300 comprises a substantially cylindrical shaped cavity configured to receive the substantially cylindrically shaped tip of the first shaft portion 212 and having a matching undulating outer profile. The second portion 320 of the implant 300 is located in the center of the substantially cylindrical shaped cavity of the first portion 310 of the implant 300 and configured to cooperate with the actuation member 120 via a matching undulating inner profile. In this example, an actuation of the actuation member 120 rotates the second portion 320 relative to the first portion 310 of the implant 300. The rotation of the second portion 320 further leads to a change of an actuation state of the implant 300, in particular to a change of dimensions (here: a height of the implant 300).

Fig. 6B shows a side view of the implant 300 with a focus on a mechanism configured to actuate (here: expand or retract) at least a part of the implant 300 relative to the first shaft portion 212 when the second portion 320 of the implant 300 is actuated by the actuation member 120. In the view of Fig. 6B, the implant 300 comprises an actuatable upper implant part 330 and an actuatable lower implant part 340 each comprising a guiding portion configured to define the direction of the actuation movement of the implant parts 330, 340 either away from each other (expansion) or towards each other (retraction). Each guiding portion comprises a slit-like guiding opening 350, 360. The guiding openings 350, 360 cooperate with a guide pin 370 extending into the guiding openings 350, 360 for guiding movements of the implant parts 330, 340.

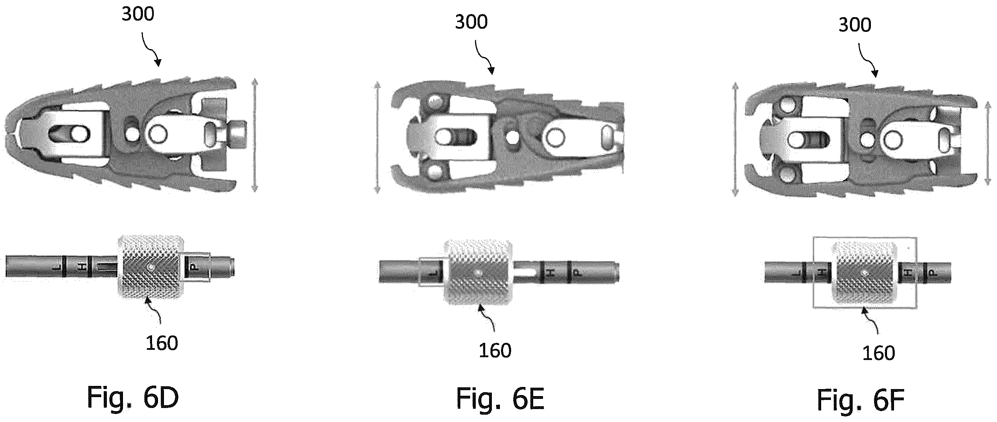

Figs. 6D to 6F each show a schematical side view of the implant 300 with a corresponding position of the drive member 160 along the longitudinal axis A_{L}. Each side view refers to a different engagement of the implant 300 with the actuation member 120 exemplarily as shown in Figs. 3A and 3B. The engagement of the implant 300 and the actuation member 120 is in each case dependent on the position of the drive member 160 along the longitudinal axis A_{L}.

In Fig. 6D, the drive member 160 is located in a first position along the longitudinal axis A_{L} and the implant 300 is configured for posterior expansion or retraction. In Fig. 6E, the drive member 160 is located in a second position along the longitudinal axis A_{L} and the implant 300 is configured for lordotical expansion or retraction. In Fig. 6F, the drive member 160 is located in a third position along the longitudinal axis A_{L} and the implant 300 is configured for a symmetric height expansion or retraction.

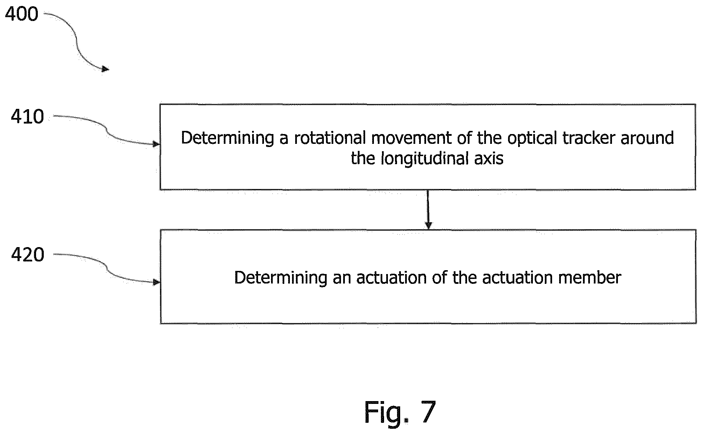

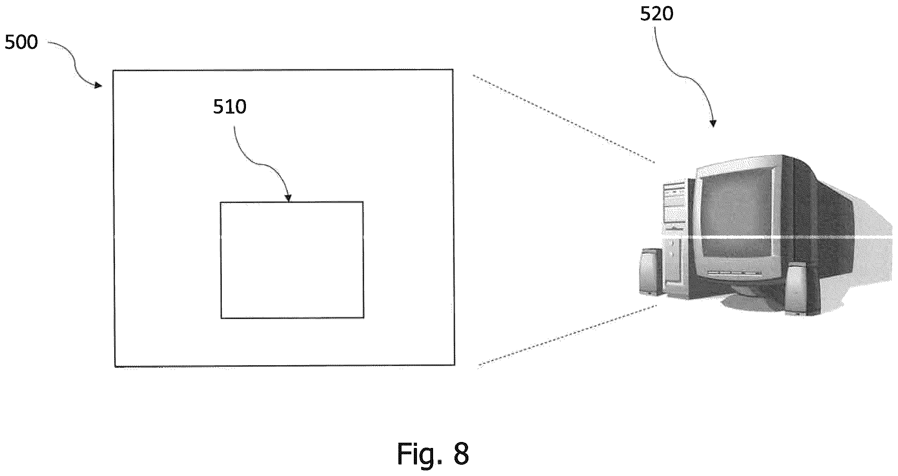

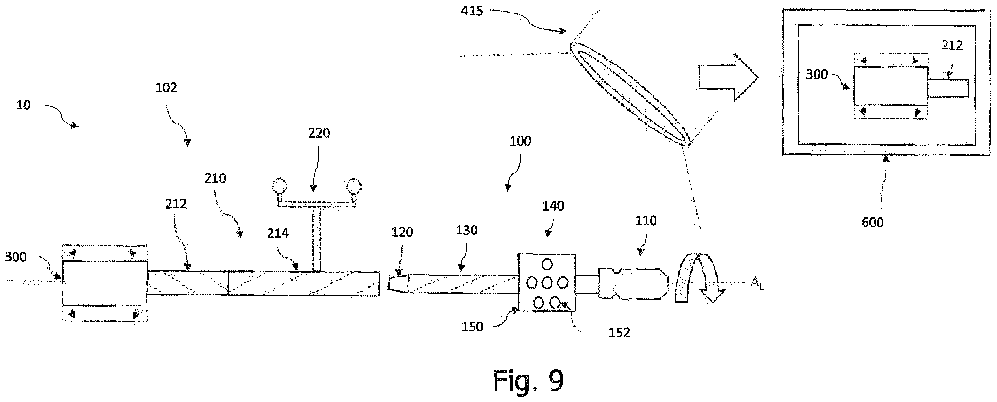

Fig. 7 illustrates in a block diagram 400 a computer-implemented method embodiment for determining an actuation of the actuation member 120 of the surgical device 10 described above. The method embodiment will be explained with reference to Figs. 8 and 9.

Fig. 8 shows a schematic representation of a computer program product 500 with code 510 executable by a processor of a computer 520. The code is configured to cause the processor to carry out the method embodiment when executed by the processor. The computer 520 may belong to a surgical navigation system as commonly used for computer assisted surgery.

Fig. 9 shows a schematic representation of such a surgical navigation system with an additional optical sensor 415 connected to a computer 520 (see Fig. 8) and a display 600 also connected to the computer 520. The surgical navigation system is configured for visualizing at least the actuatable implant 300 and, optionally, at least a part of the first shaft portion 212 on the display 600.

Referring now to Fig. 7 again, in step 410, the computer 520 determines a position of the first optical tracker 140 around the longitudinal axis A_{L} defined by the shaft 210 of the surgical device 10. In one example, determining the position may be performed automatically via the optical sensor 415 and the computer 520. Based on data from the optical sensor 415, the computer 520 in some scenarios tracks a change of the distinctive arrangement of the optically-detectable markers 152 of the first optical tracker 140 from a given viewing direction (e.g., relative to the second optical tracker 220). In this way, a positional change of the first optical tracker 140 around the longitudinal axis A_{L} is determined.

In step 420, the computer 520 determines, from at least the position of the first optical tracker 140 and optionally further information, an actuation of the actuation member 120. As an example, based on data from the optical sensor 415, the computer 520 may continuously track the distinctive arrangement of the optically-detectable markers 152 of the first optical tracker 140. Any newly received data are compared with previously received ones. The comparison may be done in real-time. If a difference between the data is determined, the first optical tracker 140 was rotated around the longitudinal axis A_{L} and consequently, the actuation member 120 was actuated. If no difference between the data can be determined, the first optical tracker 140 was not or no longer rotated and thus, the actuation member 120 was not or no longer actuated. A continuous comparison of received results therefore enables determining a start and an end of an actuation as well as determining a rotation angle covered by the first optical tracker 140 during the determined actuation (e.g., relative to the second optical tracker 220).

Additionally, an actuation state of the implant 300 resulting from the actuation can be determined by the computer 520 based on the determined angle of rotation. In particular, the resulting actuation state can be determined relative to a known initial state prior to the actuation. Therefore, the known initial actuation state can be combined with the determined angle of rotation to determine a later actuation state of the implant 300. In particular, a dimensional change of the implant 300 can be determined based on a predefined (e.g., functional) relationship between the first optical tracker 140 and the actuatable implant 300 as pre-stored by the computer 520.

For the visualization, image data of the surgical device 10 and the implant 300 may be pre-stored by the computer 520. The optical sensor 415 may track the movements of the first and the second optical trackers 140, 220 and send tracking related data to the computer 520. The computer 520 receives and processes these data and correspondingly visualizes models of the surgical device 10 and the implant 300 on the display 600.

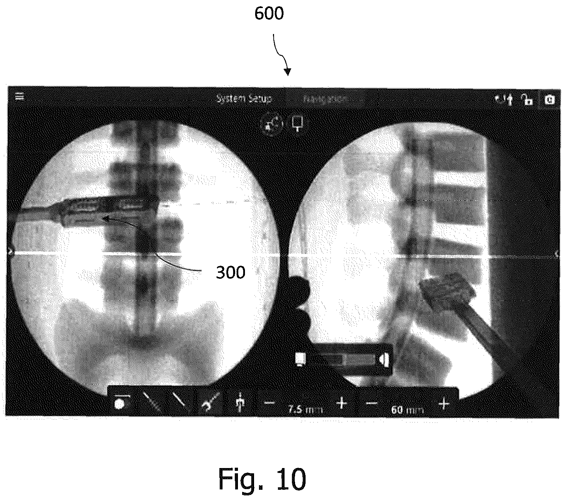

In the exemplary display view shown in Fig. 10, the visualization comprises visualizing the implant 300 in relation to registered three dimensional image data of a patient to assist a surgeon during a surgery. The image data of the patient may also be pre-stored by the computer 520. Additionally, the position of the patient may be tracked via additional optical patient trackers. A visualization in real-time may comprise tracking movement of both, the patient and the surgical device 10 and converting the tracked movement into a visualization on the display 600.

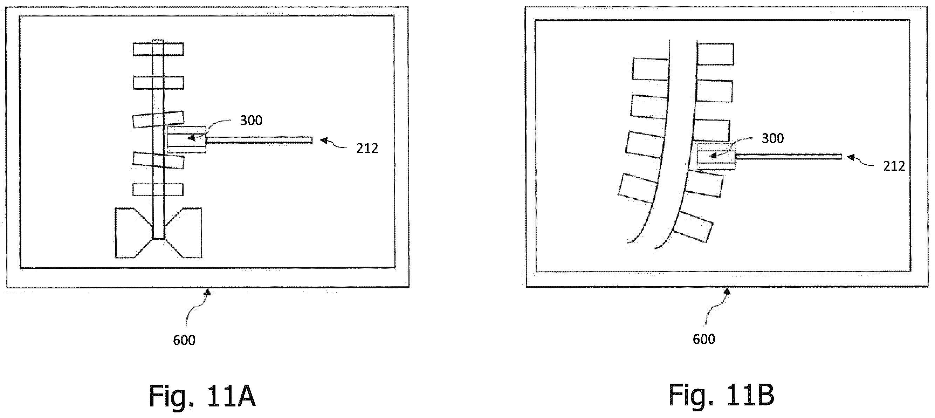

Figs. 11A and 11B show schematic representations of the visualization of the actuatable implant 300 (and, optionally, at least a part of the first shaft portion 212) with registered three dimensional image data of a patient on the display 600 for a spinal intervention. A change of a dimension of the actuatable implant 300 is also visualized in real time. For visualizing the dimensional change of the implant 300, the rotation of the optical tracker 140 is tracked by the optical sensor 415 and translated by the computer 520 according to the relationship between the rotational movement of the optical tracker 140 and the actuation of the implant 300 as described above.

Further, assuming that the drive member 160 can be tracked also (e.g., because its function is assumed by the first optical tracker 140, or because the first optical tracker 140 is rigidly attached to the drive member 160, or because a separate third optical tracker is rigidly attached thereto), the position of the drive member 160 along the longitudinal axis A_{L} may be tracked by the optical sensor 415, for example by tracking an axial distance between the first optical tracker 140 and the second optical tracker 220. The tracked position may be translated by the computer 520 into a particular engagement of the implant 300 and the actuation member 120, so as to determine the type of expansion or retraction of the implant 300 (e.g., posteriorly, lordotically or symmetric height expansion or retraction, as described above) for later visualization. Alternatively to tracking the position of the drive member 160 with the optical sensor 415, the position of the drive member 160 may be entered manually into the computer 520, for example via a graphical user interface (GUI).

Based on the results of the translation of the rotation of the optical tracker 140 and, optionally, based on the position of the drive member 160 along the longitudinal axis A_{L}, the computing system determines and applies the dimensional change on the implant model visualized on the display 600. All visualizations described above may be done in real-time.

As has become apparent from the above description of exemplary embodiments, the surgical device presented herein comprises an optical tracker that rotates relative to a longitudinal axis when the surgical device is operated. This rotation can be translated into an actuation movement of, for example, an implant. The actuation movement, in turn, can be visualized despite not being observable as such.

It will be appreciated that the present disclosure is not limited to detecting an actuation movement of an implant. Rather, the surgical device may have an actuation member that harvests tissue, or performs any other surgical task, and the state of that task can also be visualized as described above.

## Claims

1. A surgical device (10) comprising:
a shaft (210) defining a longitudinal axis (A_{L}), the shaft (210) having a first shaft portion (212) configured to be inserted into a patient and a second shaft portion (214) configured to be located outside the patient when the first shaft portion (212) is inserted in the patient;
an actuation member (120) actuatable relative to the first shaft portion (212);
an operation member (110) operable relative to the second shaft portion (214), the operation member (110) being configured to actuate the actuation member (120) when being operated; and
a first optical tracker (140) coupled with the operation member (110) so that operation of the operation member (110) causes the first optical tracker (140) to rotate relative to the longitudinal axis (A_{L}), wherein
the first optical tracker (140) comprises
a body (150) arranged or configured to be arranged co-axially to the longitudinal axis (A_{L}) of the shaft (210) and having a substantially cylindrical shape with a cylinder axis; and
a plurality of optically detectable markers (152) located on or within the body and arranged in a pattern that defines, from a given viewing direction, distinctive arrangements of the markers (152) in different rotational states of the first optical tracker (140) relative to the longitudinal axis (A_{L}), wherein at least some of the markers are spaced apart from each other in a circumferential direction around the longitudinal axis.

2. The surgical device according to any preceding claim, wherein
there exists a predefined relationship between a movement of the first optical tracker (140) on the one hand and an actuation movement of one of the actuation member (120) and an actuatable implant (300) detachably couplable to the actuation member (120) on the other hand.

3. The surgical device (10) according to claim 2, wherein
the predefined relationship associates a change of a rotational state of the first optical tracker (140) resulting from its rotation with a change of a dimension or state of one of the actuation member (120) and the actuatable implant (300) detachably couplable to the actuation member (120) resulting from the actuation movement.

4. The surgical device (10) according to any preceding claim, wherein
the operation member (110) is rotatable around the longitudinal axis (A_{L}).

5. The surgical device (10) according to any preceding claim, wherein
the first optical tracker (140) is rigidly coupled or configured to be rigidly coupled to the operation member (110).

6. The surgical device (10) according to any preceding claim, comprising
a second tracker (220) rigidly coupled or configured to be rigidly coupled to the shaft (210).

7. The surgical device (10) according to claim 6, wherein
the operation member (110) is movable relative to the shaft (210) in an axial direction relative to the longitudinal axis (A_{L}) from a predefined first axial position to at least a predefined second axial position, wherein
the surgical device (10) is configured such that an axial movement of the operation member (110) relative to the shaft (210) leads to an axial movement of the first optical tracker (140) relative to the second tracker (220).

8. The surgical device (10) according to any preceding claims, wherein
operation of the operation member (110) causes a rotation of the actuation member (120) relative to the first shaft portion (212).

9. The surgical device (10) according to any of the preceding claims, wherein
the shaft (210) defines a passage; and
the operation member (110) is coupled to the actuation member (120) via a coupling section (130) extending into the passage defined by the shaft (210).

10. The surgical device (10) according to any preceding claim, wherein
the first shaft portion (212) is configured to cooperate with a first portion of an implant (300) and the actuation member (120) is configured to cooperate with a second portion of the implant (300), wherein the second portion of the implant is movable relative to the first portion of the implant for a change of an actuation state of the implant (300).

11. The surgical device (10) according to claim 10, wherein
the shaft (210) is configured to detachably receive the implant (300) for placement of the implant (300) in the body of the patient.

12. The surgical device (10) according to any preceding claim, wherein
the first optical tracker (140) comprises
a cover layer (154) for the body (150); and
one or more optically-detectable markers (152) arranged between the body (150) and the cover layer (154), wherein the cover layer is optically transparent at least in one or more regions where the one or more optically-detectable markers (152) are located.

13. A system comprising the surgical device (10) of any of claims 1 to 12 and an implant (300) configured to be actuated by the actuation member (120) from a first actuation state to a second actuation state.

14. The system according to claim 13, wherein
the implant (300) is configured to change at least one dimension when being actuated from the first actuation state to the second actuation state.

15. The system according to claim 13 or 14, wherein
at least a part of the implant (300) is configured to extend relative to the first shaft portion (212) when the implant (300) is actuated from the first actuation state to the second actuation state; or
at least a part of the implant (300) is configure to retract when the implant (300) is actuated from the second actuation state to the first actuation state.

16. A computer program product (500) with program code (510) for carrying out a computer-implemented method (400) for determining actuation performed with a surgical device (10), the surgical device (10) comprising:
a shaft (210) defining a longitudinal axis (A_{L}), the shaft (210) having a first shaft portion (212) configured to be inserted into a patient and a second shaft portion (214) configured to be located outside the patient when the first shaft portion (212) is inserted in the patient;
an actuation member (120) actuatable relative to the first shaft portion (212);
an operation member (110) operable relative to the second shaft portion (214), the operation member (110) being configured to actuate the actuation member (120) when being operated; and
a first optical tracker (140) coupled with the operation member (110) so that operation of the operation member (110) causes the first optical tracker (140) to rotate relative to the longitudinal axis (A_{L}), wherein
the first optical tracker (140) comprises a plurality of markers (152) arranged in a pattern that defines, from a given viewing direction, distinctive arrangements of the markers (152) in different rotational states of the first optical tracker (140) relative to the longitudinal axis (A_{L});
the method (400) comprising:
determining (410) a rotational state of the first optical tracker (140);
determining (420), from at least the rotational state of the first optical tracker (140), an actuation of at least one of the actuation member (120) and an implant (300) detachably couplable to the actuation member (120).

17. The computer program product (500) according to claim 16, wherein the method (400) further comprises:
determining an actuation state of the actuation member (120) resulting from the actuation relative to a known initial state prior to the actuation.

18. The computer program product (500) according to claim 16 or 17, wherein
the position of the first optical tracker (140) is determined relative to a second tracker (220) having a fixed relationship relative to the shaft (210) by tracking the first and second trackers (140; 220).

19. The computer program product (500) according to any one of claims 16 to 18, wherein the method (400) further comprises:
visualizing the actuation of the actuation member (120) on a display (600), wherein, optionally, visualizing of the actuation of the actuation member (120) is performed in real-time relative to an image of the patient.

## Patentansprüche

1. Chirurgische Vorrichtung (10), umfassend:
einen Schaft (210), der eine Längsachse (A_{L}) definiert, wobei der Schaft (210) einen ersten Schaftabschnitt (212), der dazu eingerichtet ist, in einen Patienten eingeführt zu werden, und einen zweiten Schaftabschnitt (214) aufweist, der dazu eingerichtet ist, sich außerhalb des Patienten zu befinden, wenn der erste Schaftabschnitt (212) in den Patienten eingeführt ist;
ein Betätigungselement (120), das relativ zum ersten Schaftabschnitt (212) betätigbar ist;
ein Bedienelement (110), das relativ zum zweiten Schaftabschnitt (214) bedienbar ist, wobei das Bedienelement (110) dazu eingerichtet ist, das Betätigungselement (120) zu betätigen, wenn es bedient wird; und
einen ersten optischen Tracker (140), der mit dem Bedienelement (110) gekoppelt ist, sodass eine Bedienung des Bedienelements (110) bewirkt, dass sich der erste optische Tracker (140) relativ zur Längsachse (A_{L}) dreht, wobei
der erste optische Tracker (140) umfasst:
einen Körper (150), der koaxial zur Längsachse (A_{L}) des Schafts (210) angeordnet oder dazu eingerichtet ist, derart angeordnet zu werden, und eine im Wesentlichen zylindrische Form mit einer Zylinderachse aufweist; und
eine Mehrzahl von optisch detektierbaren Markierungen (152), die sich auf oder in dem Körper befinden und in einem Muster angeordnet sind, das aus einer gegebenen Betrachtungsrichtung charakteristische Anordnungen der Markierungen (152) in verschiedenen Drehzuständen des ersten optischen Trackers (140) relativ zur Längsachse (A_{L}) definiert, wobei mindestens einige der Markierungen in einer Umfangsrichtung um die Längsachse voneinander beabstandet sind.

2. Chirurgische Vorrichtung nach einem der vorhergehenden Ansprüche, wobei
eine vordefinierte Beziehung existiert zwischen einer Bewegung des ersten optischen Trackers (140) einerseits und einer Betätigungsbewegung entweder des Betätigungselements (120) oder eines betätigbaren Implantats (300), das lösbar mit dem Betätigungselement (120) koppelbar ist, andererseits.

3. Chirurgische Vorrichtung (10) nach Anspruch 2, wobei
die vordefinierte Beziehung eine Änderung eines Drehzustands des ersten optischen Trackers (140), die aus seiner Drehung resultiert, mit einer Änderung einer Abmessung oder eines Zustands entweder des Betätigungselements (120) oder des betätigbaren Implantats (300), das lösbar mit dem Betätigungselement (120) koppelbar ist, die aus der Betätigungsbewegung resultiert, verknüpft.

4. Chirurgische Vorrichtung (10) nach einem der vorhergehenden Ansprüche, wobei
das Bedienelement (110) um die Längsachse (A_{L}) drehbar ist.

5. Chirurgische Vorrichtung (10) nach einem der vorhergehenden Ansprüche, wobei
der erste optische Tracker (140) starr mit dem Bedienelement (110) gekoppelt oder dazu eingerichtet ist, starr mit diesem gekoppelt zu werden.

6. Chirurgische Vorrichtung (10) nach einem der vorhergehenden Ansprüche, umfassend
einen zweiten Tracker (220), der starr mit dem Schaft (210) gekoppelt oder dazu eingerichtet ist, starr mit diesem gekoppelt zu werden.

7. Chirurgische Vorrichtung (10) nach Anspruch 6, wobei
das Bedienelement (110) relativ zum Schaft (210) in einer axialen Richtung relativ zur Längsachse (A_{L}) von einer vordefinierten ersten axialen Position zu mindestens einer vordefinierten zweiten axialen Position bewegbar ist, wobei
die chirurgische Vorrichtung (10) so eingerichtet ist, dass eine axiale Bewegung des Bedienelements (110) relativ zum Schaft (210) zu einer axialen Bewegung des ersten optischen Trackers (140) relativ zum zweiten Tracker (220) führt.

8. Chirurgische Vorrichtung (10) nach einem der vorhergehenden Ansprüche, wobei
eine Bedienung des Bedienelements (110) eine Drehung des Betätigungselements (120) relativ zum ersten Schaftabschnitt (212) bewirkt.

9. Chirurgische Vorrichtung (10) nach einem der vorhergehenden Ansprüche, wobei
der Schaft (210) einen Durchgang definiert; und
das Bedienelement (110) über einen Kopplungsabschnitt (130), der sich in den durch den Schaft (210) definierten Durchgang erstreckt, mit dem Betätigungselement (120) gekoppelt ist.

10. Chirurgische Vorrichtung (10) nach einem der vorhergehenden Ansprüche, wobei
der erste Schaftabschnitt (212) dazu eingerichtet ist, mit einem ersten Abschnitt eines Implantats (300) zusammenzuwirken, und das Betätigungselement (120) dazu eingerichtet ist, mit einem zweiten Abschnitt des Implantats (300) zusammenzuwirken, wobei der zweite Abschnitt des Implantats relativ zum ersten Abschnitt des Implantats für eine Änderung eines Betätigungszustands des Implantats (300) bewegbar ist.

11. Chirurgische Vorrichtung (10) nach Anspruch 10, wobei
der Schaft (210) dazu eingerichtet ist, das Implantat (300) zur Platzierung des Implantats (300) im Körper des Patienten lösbar aufzunehmen.

12. Chirurgische Vorrichtung (10) nach einem der vorhergehenden Ansprüche, wobei
der erste optische Tracker (140) umfasst:
eine Deckschicht (154) für den Körper (150); und
eine oder mehrere optisch detektierbare Markierungen (152), die zwischen dem Körper (150) und der Deckschicht (154) angeordnet sind, wobei die Deckschicht mindestens in einem oder mehreren Bereichen, in denen sich die eine oder die mehreren optisch detektierbaren Markierungen (152) befinden, optisch transparent ist.

13. System, umfassend die chirurgische Vorrichtung (10) nach einem der Ansprüche 1 bis 12 und ein Implantat (300), das dazu eingerichtet ist, durch das Betätigungselement (120) von einem ersten Betätigungszustand in einen zweiten Betätigungszustand betätigt zu werden.

14. System nach Anspruch 13, wobei
das Implantat (300) dazu eingerichtet ist, mindestens eine Abmessung zu ändern, wenn es vom ersten Betätigungszustand in den zweiten Betätigungszustand betätigt wird.

15. System nach Anspruch 13 oder 14, wobei
mindestens ein Teil des Implantats (300) dazu eingerichtet ist, sich relativ zum ersten Schaftabschnitt (212) auszudehnen, wenn das Implantat (300) vom ersten Betätigungszustand in den zweiten Betätigungszustand betätigt wird; oder
mindestens ein Teil des Implantats (300) dazu eingerichtet ist, sich zurückzuziehen, wenn das Implantat (300) vom zweiten Betätigungszustand in den ersten Betätigungszustand betätigt wird.

16. Computerprogrammprodukt (500) mit Programmcode (510) zur Ausführung eines computerimplementierten Verfahrens (400) zum Bestimmen einer mit einer chirurgischen Vorrichtung (10) durchgeführten Betätigung, wobei die chirurgische Vorrichtung (10) Folgendes umfasst:
einen Schaft (210), der eine Längsachse (A_{L}) definiert, wobei der Schaft (210) einen ersten Schaftabschnitt (212), der dazu eingerichtet ist, in einen Patienten eingeführt zu werden, und einen zweiten Schaftabschnitt (214) aufweist, der dazu eingerichtet ist, sich außerhalb des Patienten zu befinden, wenn der erste Schaftabschnitt (212) in den Patienten eingeführt ist;
ein Betätigungselement (120), das relativ zum ersten Schaftabschnitt (212) betätigbar ist;
ein Bedienelement (110), das relativ zum zweiten Schaftabschnitt (214) bedienbar ist, wobei das Bedienelement (110) dazu eingerichtet ist, das Betätigungselement (120) zu betätigen, wenn es bedient wird; und
einen ersten optischen Tracker (140), der mit dem Bedienelement (110) gekoppelt ist, sodass eine Bedienung des Bedienelements (110) bewirkt, dass sich der erste optische Tracker (140) relativ zur Längsachse (A_{L}) dreht, wobei der erste optische Tracker (140) eine Mehrzahl von Markierungen (152) umfasst, die in einem Muster angeordnet sind, das aus einer gegebenen Betrachtungsrichtung charakteristische Anordnungen der Markierungen (152) in verschiedenen Drehzuständen des ersten optischen Trackers (140) relativ zur Längsachse (A_{L}) definiert;
wobei das Verfahren (400) Folgendes umfasst:
Bestimmen (410) eines Drehzustands des ersten optischen Trackers (140);
Bestimmen (420), aus mindestens dem Drehzustand des ersten optischen Trackers (140), einer Betätigung des Betätigungselements (120) und/oder eines Implantats (300), das lösbar mit dem Betätigungselement (120) koppelbar ist.

17. Computerprogrammprodukt (500) nach Anspruch 16, wobei das Verfahren (400) weiterhin Folgendes umfasst:
Bestimmen eines Betätigungszustands des Betätigungselements (120), der aus der Betätigung resultiert, relativ zu einem bekannten Anfangszustand vor der Betätigung.

18. Computerprogrammprodukt (500) nach Anspruch 16 oder 17, wobei
die Position des ersten optischen Trackers (140) relativ zu einem zweiten Tracker (220) bestimmt wird, der eine feste Beziehung relativ zum Schaft (210) aufweist, indem der erste und der zweite Tracker (140; 220) verfolgt werden.

19. Computerprogrammprodukt (500) nach einem der Ansprüche 16 bis 18, wobei das Verfahren (400) weiterhin Folgendes umfasst:
Visualisieren der Betätigung des Betätigungselements (120) auf einer Anzeige (600), wobei das Visualisieren der Betätigung des Betätigungselements (120) optional in Echtzeit relativ zu einem Bild des Patienten durchgeführt wird.

## Revendications

1. Dispositif chirurgical (10) comprenant:
une tige (210) définissant un axe longitudinal (A_{L}), la tige (210) comportant une première partie tige (212) conçue pour être insérée dans un patient et une deuxième partie tige (214) conçue pour se trouver à l'extérieur du patient lorsque la première partie tige (212) est insérée dans le patient;
un élément d'actionnement (120) pouvant être actionné par rapport à la première partie tige (212);
un élément de commande (110) pouvant être actionné par rapport à la deuxième partie tige (214), l'élément de commande (110) étant conçu pour actionner l'élément d'actionnement (120) lorsqu'il est actionné; et
un premier dispositif de suivi optique (140) couplé à l'élément de commande (110) de telle sorte que l'actionnement de l'élément de commande (110) amène le premier dispositif de suivi optique (140) à tourner par rapport à l'axe longitudinal (A_{L}), dans lequel
le premier dispositif de suivi optique (140) comprend
un corps (150) agencé ou conçu pour être disposé coaxialement à l'axe longitudinal (A_{L}) de la tige (210) et présentant une forme sensiblement cylindrique avec un axe de cylindre; et
une pluralité de repères optiquement détectables (152) situés sur ou à l'intérieur du corps et disposés selon un motif qui définit, à partir d'une direction d'observation donnée, des agencements distinctifs des repères (152) dans différents états de rotation du premier dispositif de suivi optique (140) par rapport à l'axe longitudinal (AL), dans lequel au moins certains des repères sont espacés les uns des autres dans une direction circonférentielle autour de l'axe longitudinal.

2. Dispositif chirurgical selon l'une quelconque revendication précédente, dans lequel
il existe une relation prédéfinie entre, d'une part, un mouvement du premier dispositif de suivi optique (140) et, d'autre part, un mouvement d'actionnement de l'un parmi l'élément d'actionnement (120) ou un implant actionnable (300) pouvant être couplé de manière amovible à l'élément d'actionnement (120).

3. Dispositif chirurgical (10) selon la revendication 2, dans lequel
la relation prédéfinie associe un changement de rotation du premier dispositif de suivi optique (140), résultant de sa rotation, à une variation de la dimension ou de l'état de l'un parmi l'élément d'actionnement (120) ou l'implant actionnable (300) pouvant être couplé de manière amovible à l'élément d'actionnement (120), obtenue par le mouvement d'actionnement.

4. Dispositif chirurgical (10) selon l'une quelconque de revendication, dans lequel
l'élément de commande (110) peut tourner autour de l'axe longitudinal (A_{L}).

5. Dispositif chirurgical (10) selon l'une quelconque de revendication, dans lequel
le premier dispositif de suivi optique (140) est solidarisé ou est conçu pour être solidarisé à l'élément de commande (110).

6. Dispositif chirurgical (10) selon l'une quelconque de revendication, dans lequel
un deuxième dispositif de suivi (220) est solidarisé ou est conçu pour être solidarisé à la tige (210).

7. Dispositif chirurgical (10) selon la revendication 6, dans lequel
l'élément de commande (110) est mobile par rapport à la tige (210) dans une direction axiale par rapport à l'axe longitudinal (A_{L}), depuis une première position axiale prédéfinie jusqu'à au moins une deuxième position axiale prédéfinie, dans lequel
le dispositif chirurgical (10) est conçu de telle sorte qu'un mouvement axial de l'élément de commande (110) par rapport à la tige (210) entraîne un mouvement axial du premier suiveur optique (140) par rapport au deuxième suiveur (220).

8. Dispositif chirurgical (10) selon l'une quelconque des revendication précédentes, dans lequel
la commande de l'élément de commande (110) entraîne une rotation de l'élément d'actionnement (120) par rapport à la première partie tige (212).

9. Dispositif chirurgical (10) selon l'une quelconque des revendications précédentes, dans lequel
la tige (210) délimite un passage; et
l'élément de commande (110) est relié à l'élément d'actionnement (120) par l'intermédiaire d'une section de liaison (130) s'étendant dans le passage délimité par la tige (210)

10. Dispositif chirurgical (10) selon l'une quelconque revendication précédente, dans lequel
la première partie tige (212) est conçue pour coopérer avec une première partie d'un implant (300) et l'élément d'actionnement (120) est conçu pour coopérer avec une deuxième partie de l'implant (300), la deuxième partie de l'implant pouvant se déplacer par rapport à la première partie de l'implant afin de modifier l'état d'actionnement de l'implant (300).

11. Dispositif chirurgical (10) selon la revendication 10, dans lequel
la tige (210) est conçue pour recevoir de manière amovible l'implant (300) en vue de la mise en place de l'implant (300) dans le corps du patient.

12. Dispositif chirurgical (10) selon l'une quelconque revendication précédente, dans lequel
le premier dispositif de suivi optique (140) comprend
une couche de recouvrement (154) pour le corps (150); et
un ou plusieurs marqueurs détectables optiquement (152) disposés entre le corps (150) et la couche de recouvrement (154), la couche de recouvrement étant optiquement transparente au moins dans une ou plusieurs zones où se trouvent le ou les marqueurs détectables optiquement (152).

13. Système comprenant le dispositif chirurgical (10) selon l'une quelconque des revendications 1 à 12 et un implant (300) conçu pour être actionné par l'élément d'actionnement (120) d'une première position d'actionnement à une seconde position d'actionnement.

14. Système selon la revendication 13, dans lequel
l'implant (300) est conçu pour modifier au moins une dimension lorsqu'il passe du premier état d'actionnement au deuxième état d'actionnement.

15. Système selon la revendication 13 ou 14, dans lequel
au moins une partie de l'implant (300) est conçue pour s'étendre par rapport à la première partie tige (212) lorsque l'implant (300) est actionné pour passer du premier état d'actionnement au deuxième état d'actionnement; ou
au moins une partie de l'implant (300) est conçue pour se rétracter lorsque l'implant (300) passe du deuxième état d'actionnement au premier état d'actionnement.

16. Produit programme informatique (500) comprenant un code de programme (510) destiné à exécuter un procédé mis en œuvre par ordinateur (400) pour déterminer l'actionnement effectué à l'aide d'un dispositif chirurgical (10), le dispositif chirurgical (10) comprenant:
une tige (210) définissant un axe longitudinal (AL), la tige (210) comportant une première partie tige (212) conçue pour être insérée dans un patient et une deuxième partie tige (214) conçue pour se trouver à l'extérieur du patient lorsque la première partie tige (212) est insérée dans le patient;
un élément d'actionnement (120) pouvant être actionné par rapport à la première partie tige (212);
un élément de commande (110) pouvant être utilisé par rapport à la deuxième partie tige (214), l'élément de commande (110) étant conçu pour actionner l'élément d'actionnement (120) lorsqu'il est manié; et
un premier dispositif de suivi optique (140) couplé à l'élément de commande (110) de telle sorte que l'actionnement de l'élément de commande (110) amène le premier dispositif de suivi optique (140) à tourner par rapport à l'axe longitudinal (A_{L}), dans lequel
le premier dispositif de suivi optique (140) comprend une pluralité de repères optiquement détectables (152) disposés dans un motif qui définit, à partir d'une direction de visualisation, différents agencements des repères (152) dans différents états de rotation du premier dispositif de suivi optique (140) par rapport à l'axe longitudinal (A_{L}),
le procédé (400) comprenant:
la détermination (410) d'une rotation du premier dispositif de suivi (140);
la détermination (420), à partir au moins de la rotation du premier dispositif de suivi optique (140), de l'actionnement d'au moins l'un parmi l'élément d'actionnement (120) et un implant (300) pouvant être couplé de manière amovible à l'élément d'actionnement (120).

17. Produit programme informatique (500) selon la revendication 16, dans lequel le procédé (400) comprenant en outre:
la détermination d'un actionnement de l'élément d'actionnement (120) obtenue par la rotation par rapport à un état initial précédant l'actionnement.

18. Produit programme informatique (500) selon la revendication 16 ou 17, dans lequel
la position du premier dispositif de suivi optique (140) est déterminée par rapport à un deuxième dispositif de suivi (220) dont la relation est fixe par rapport à la tige (210), en effectuant un suivi des premier et deuxième dispositifs de suivi (140; 220).

19. Produit programme informatique (500) selon l'une quelconque des revendications 16 à 18, dans lequel le procédé (400) comprenant en outre:
l'affichage de l'actionnement de l'élément d'actionnement (120) sur un écran (600), l'affichage de l'actionnement de l'élément d'actionnement (120) pouvant, éventuellement, être effectué en temps réel par rapport à une image du patient.
